# EUROPEAN PATENT APPLICATION

(11) **EP 4 197 595 A1**
(43) Date of publication of application: **21.06.2023**
(21) Application number: 22200583.7
(22) Date of filing: 10.10.2022
(51) Int. Cl.: A61P 9/00, G01N 33/68

(54) **USE OF BIOMARKER OR BIOMARKERS COMBINATION IN PREPARING A DIAGNOSTIC REAGENT FOR FULMINANT MYOCARDITIS AND A DRUG FOR FULMINANT MYOCARDITIS**

(30) Priority: 20.12.2021 CN 202111559565
(71) Applicant: Tongji Hospital Tongji Medical College Huazhong University of Science and Technology, Hubei 430030 (CN)
(72) Inventor: Wang, Daowen, Wuhan City (CN); Chen, Chen, Wuhan City (CN); Jiang, Jiangang, Wuhan City (CN); Wang, Jin, Wuhan City (CN); Zhuang, Yan, Wuhan City (CN); Li, Huihui, Wuhan City (CN); Chen, Yanghui, Wuhan City (CN); He, Mengying, Wuhan City (CN)
(74) Representative: Grünecker Patent- und Rechtsanwälte PartG mbB

(57) **Abstract**

The invention "use of biomarker or biomarkers combination in preparing a diagnostic reagent for fulminant myocarditis and a drug for fulminant myocarditis" belongs to the field of disease diagnostic reagents and drugs. The use is characterized in that the biomarker comprises Siglec-5. The biomarkers combination is further selected from the group consisting of sST2, PAI-1, Siglec-5, CD163, CD40, P-Cadherin, CD14, CTLA4. The invention finds that the levels of the biomarker in plasma of patients with fulminant myocarditis is increased, and proves that the biomarker plays a role in diagnosing fulminant myocarditis and a role as a drug target in relieving and/or improving fulminant myocarditis. The level of the biomarker can be detected to forecast or auxiliary diagnose fulminant myocarditis or estimate prognosis of fulminant myocarditis. Meanwhile, level of the biomarkers is controlled through drugs, more intensified diagnosis and follow-up treatment can be carried out on diseases, and the biomarkers have huge clinical application value.

## Description

### Technical field

The invention belongs to the field of disease diagnostic reagents and drugs, and specifically relates to use of biomarker or biomarkers combination in preparing a diagnostic reagent for fulminant myocarditis and a drug for fulminant myocarditis.

### Technical background

Fulminant myocarditis is the most serious and special type of myocarditis, whose main features are sudden onset, extremely rapid progression, and rapid hemodynamic abnormalities rapidly happened in patients who are accompanied by respiratory failure and liver and kidney failure, with an early mortality rate of more than 50%.

The clinical characteristics as well as diagnosis and treatment status of fulminant myocarditis have the following features:
1. The serious lack of clinical understanding and diagnostic ability of fulminant myocarditis leads to a high rate of missed diagnosis and misdiagnosis in such patients, and treatment is often delayed.
2. There are no effective treatment protocols, consensus or guidelines worldwide. In the past, most of fulminant myocarditis has been treated empirically, including anti-infection, raising blood pressure, cardiac artificial assistance device, etc., but the survival rate of patients has not been substantially improved.
3. The effective treatment of fulminant myocarditis has great medical and social value. Fulminant myocarditis usually occurs in healthy young adults without underlying diseases. Due to the high early mortality, fulminant myocarditis causes great harm to the patients families and society.

However, it's worth noting that, once fulminant myocarditis patients survive through the risk period, their long-term survival and quality of life are almost no different from those of the general population. An 11-year follow-up study showed that the survival rate of fulminant myocarditis was significantly higher than that of common acute myocarditis.

Fulminant myocarditis has features of extremely dangerous clinical onset, rapid progression and high mortality. Previous treatment focused on enhancing myocardial contraction and increasing blood pressure, but failed to effectively reduce mortality. The fundamental reason is the lack of correct understanding of the pathophysiology and pathogenesis of fulminant myocarditis, or even the completely wrong understanding, therefore doctors cannot put forward effective treatment methods, and lead to serious consequences of continuous progress or death of patients with fulminant myocarditis. Therefore, it is urgent to fully study and understand the pathogenesis of fulminant myocarditis, find new therapeutic targets and adopt more effective measures to improve the treatment of fulminant myocarditis, and reduce mortality and hospitalization rate of fulminant myocarditis. On the other hand, diagnosis of fulminant myocarditis still lacks specific indicators, so it is necessary to find new biomarkers.

### Summary of invention

In view of problems of prior art that diagnosis and treatment of fulminant myocarditis are difficult, and risk assessment is insufficient, this invention finds a series of new biomarkers, and proves their correlations with the onset of fulminant myocarditis, and two of these (sST2 and CD163) as drug targets in reducing and/or improving function of fulminant myocarditis. Therefore, level of inflammatory factors detected by kit can be used to assist in diagnosis of fulminant myocarditis or evaluate the prognosis of fulminant myocarditis, and drugs targeting sST2 or CD163 may be with great clinical application value.

Use of biomarker or biomarkers combination in preparing diagnostic reagents for fulminant myocarditis, characterized in that, the biomarker comprises: Siglec-5.

The biomarkers combination is selected from the group consisting of sST2, PAI-1, Siglec-5, CD163, CD40, P-Cadherin, CD14, CTLA4.

Specifically, the biomarkers combination is selected from one or more of sST2, PAI-1, Siglec-5, CD163, CD40, P-Cadherin, CD14, CTLA4.

More specifically, the biomarkers combinations are selected from one, two, three, four, five, six, seven, or eight of sST2, PAI-1, Siglec-5, CD163, CD40, P-Cadherin, CD14, CTLA4.

The diagnostic reagents for fulminant myocarditis include the biomarkers combination and other diagnostically acceptable auxiliary reagents; the diagnostically acceptable auxiliary reagents include: ELISA test reagents, or flow detection reagents.

The diagnosis includes determining whether suffer from fulminant myocarditis, or assessing prognosis of fulminant myocarditis;
preferably, the diagnosis refers to whether suffer from fulminant myocarditis, or prognosis of fulminant myocarditis is assessed based on expression level of biomarker in the biomarkers combination in subject;
preferably, when Siglec-5 expression level ≥89.56 pg/mL,
and/or, sST2 expression level ≥21.39 ng/mL,
and/or, PAI-1expression level ≥41.29 ng/mL,
and/or, CD163 expression level ≥ 133.8 ng/mL,
and/or, CD40 expression level ≥67.43 pg/mL,
and/or, P-Cadherin expression level ≥2634 pg/mL,
and/or, CD14 expression level ≥262.4 ng/mL,
and/or, CTLA4 expression level ≥11.19 pg/mL, fulminant myocarditis is diagnosed;
any one of above 8 biomarkers can be used for diagnosis of fulminant myocarditis; if expression level of multiple markers met above conditions at the same time, accuracy of diagnosis is higher.
preferably, when Siglec-5 expression level < 89.56 pg/mL,
and/or, sST2 expression level < 21.39 ng/mL,
and/or, CD163 expression level < 133.8 ng/mL, prognosis of fulminant myocarditis is good.

The fulminant myocarditis is selected from fulminant myocarditis caused or triggered by viruses, bacteria, allergies, drugs, upper respiratory tract infections, and/or, intestinal infection.

Use of biomarker or biomarkers combination in preparing drug to prevent and cure fulminant myocarditis, characterized in that, the biomarker comprises: Siglec-5.

The biomarkers combination is selected from the group consisting of sST2, PAI-1, Siglec-5, CD163, CD40, P-Cadherin, CD14, CTLA4.

Specifically, the biomarkers combination is selected from one or more of sST2, PAI-1, Siglec-5, CD163, CD40, P-Cadherin, CD14, CTLA4;

More specifically, the biomarkers combination is selected from one, two, three, four, five, six, seven, or eight of sST2, PAI-1, Siglec-5, CD163, CD40, P-Cadherin, CD14, CTLA4.

Drug target of the drug to prevent and cure fulminant myocarditis is biomarker in the biomarkers combination.

Preferably, the drug to prevent and cure fulminant myocarditis includes an active ingredient that reduces, downregulates, or inhibits the biomarker.

Preferably, the active ingredient that reduces, downregulates or inhibits the biomarker is selected from inhibitor, neutralizing antibody or polypeptide of the biomarker.

The drug to prevent and cure fulminant myocarditis takes the biomarker in the biomarkers combination as the drug target, and achieves the effect of preventing and curing fulminant myocarditis by inhibiting expression level of the biomarker.

Preferably, the drug to prevent and cure fulminant myocarditis achieves effect of preventing and curing fulminant myocarditis by reducing expression level of biomarker in plasma of patients with fulminant myocarditis by inhibitor, neutralizing antibody or peptide against the biomarker.

Preferably, the fulminant myocarditis is defined as fulminant myocarditis caused or triggered by viruses, bacteria, allergies, drugs, upper respiratory tract infections, and/or intestinal infections. The drug to prevent and cure fulminant myocarditis also includes pharmaceutically acceptable excipients.

Purpose 1 of the present invention is to provide biomarkers of inflammatory factors for diagnosis of fulminant myocarditis and/or assessment of prognosis of fulminant myocarditis.

Purpose 2 of the present invention is to provide use of the biomarkers in the diagnosis of fulminant myocarditis and/or assessment of prognosis of fulminant myocarditis.

Purpose 3 of the invention is to provide above biomarkers as a novel therapeutic target of fulminant myocarditis, which achieves effect of alleviating and/or improving fulminant myocarditis by inhibiting the sST2 level of in plasma of patients with fulminant myocarditis, for example.

The technical purpose of this invention is achieved by the following technical solution:
Purpose 1 of the present invention is to provide a biomarker for diagnosis of fulminant myocarditis and/or assessment of prognosis of fulminant myocarditis. The biomarker includes sST2, PAI-1, Siglec-5, CD163, CD40, P-Cadherin, CD14 and CTLA4.

Furthermore, the fulminant myocarditis includes the fulminant myocarditis caused by various causes like viruses, bacteria, allergies, drugs, and etc.

Purpose 2 of the present invention is to provide use of biomarker in preparing reagents or kits for diagnosis of fulminant myocarditis and/or prognostic assessment of fulminant myocarditis. Furthermore, by detecting the biomarker levels in plasma of human subjects, if the biomarker concentration in plasma of human subjects is significantly increased, the human subjects are considered to have fulminant myocarditis and/or have a poor prognosis.

In this invention, through a large sample clinical trial, it was found that the levels of above inflammatory factors in plasma of patients with fulminant myocarditis were significantly increased, which confirmed that these inflammatory factors could be used as biomarkers for diagnosis of fulminant myocarditis. Therefore, detection of levels of inflammatory cytokines in plasma can be used to assist in diagnosis of fulminant myocarditis or to assess prognosis of fulminant myocarditis. Purpose 3 of the present invention is to provide use of biomarkers as therapeutic targets in preparing drugs to alleviate and/or improve fulminant myocarditis, for example, effect of alleviating and/or improving fulminant myocarditis is achieved through inhibiting the increased level of biomarkers in plasma of patients with fulminant myocarditis.

Furthermore, biomarker neutralizing antibodies are used to reduce level of biomarkers in plasma of fulminant myocarditis model mice, so as to reduce level of biomarkers in plasma of fulminant myocarditis model mice, and then achieve effect of alleviating and/or improving fulminant myocarditis.

The present invention discloses expression profile of a variety of inflammatory factors in plasma of patients with fulminant myocarditis, especially use of biomarker (Suppression of Tumorigenicity 2) and neutralizing antibodies of biomarker in fulminant myocarditis. It specifically involves use of a variety of inflammatory factors in the diagnosis and prognosis assessment of fulminant myocarditis, and use of neutralizing antibodies in treatment of fulminant myocarditis, which belongs to the field of diagnosis, prevention and treatment of cardiovascular diseases.

The invention detected expression profiles of 122 inflammatory factors in a large sample of clinical patients with fulminant myocarditis, screens out 8 inflammatory factors, and tests their efficacy as biological markers of fulminant myocarditis. The effects of neutralizing antibodies against biomarkers were further tested in fulminant myocarditis model animals, and use of biomarkers in fulminant myocarditis was determined, which provided theoretical and evidence-based basis for relief and/or treatment of fulminant myocarditis.

Technical effects of this invention are as follows:
1. The invention has confirmed correlation between specific inflammatory factors and fulminant myocarditis through a large number of experiments, and levels of various inflammatory factors are significantly increased in patients with fulminant myocarditis, suggesting that they can be used as biomarkers. Therefore, reagents or kits for detecting biomarkers are prepared. it's available to assist in diagnosis of fulminant myocarditis or evaluate prognosis of fulminant myocarditis by detecting levels of specific inflammatory markers in vitro plasma, and detection means are fast and convenient, which greatly improves efficiency and accuracy of fulminant myocarditis diagnosis and prognostic evaluation, in order to make more intensive diagnosis and subsequent treatment to the disease, and has great application value in clinic.
2. The invention not only provides a new way of diagnosis for fulminant myocarditis, but also provide a new therapeutic target of fulminant myocarditis. Biomarkers neutralizing antibody or inhibitors lower levels of biomarkers in plasma of patients with fulminant myocarditis, improve myocardial injury of fulminant myocarditis by inhibiting biomarkers. It is helpful to alleviate and improve fulminant myocarditis and greatly reduce in-hospital mortality of patients with fulminant myocarditis.

### Description of Drawings

In order to more clearly state the technical solution in embodiments of this invention, a brief description of drawings required for descriptions of the embodiments is given below, clearly, drawings referring to description below are just some examples of the present invention. For a person skilled in the art, other relevant drawings can be obtained from these drawings without creative works.

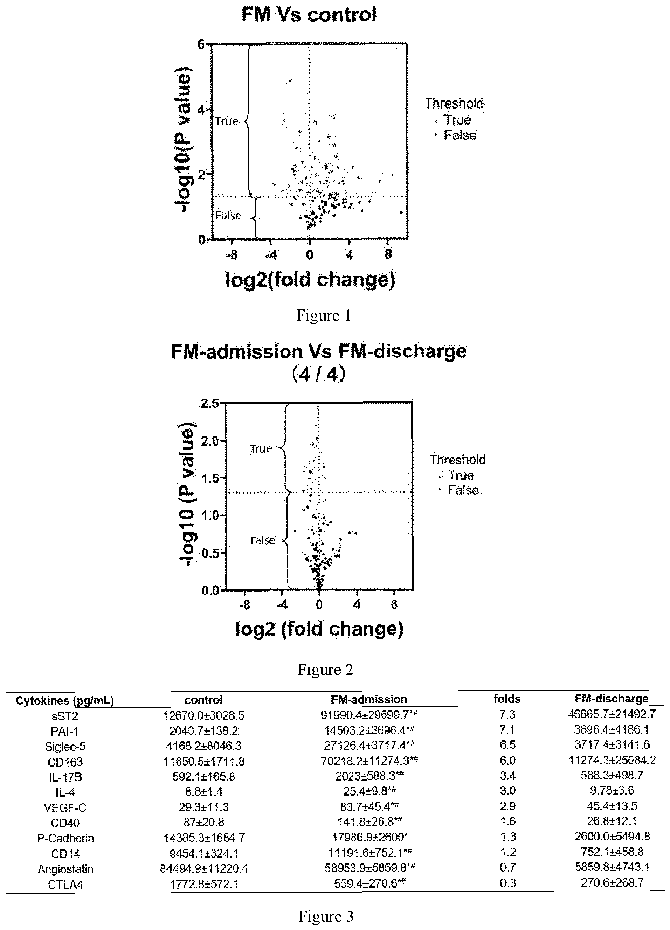

Figure 1 shows expression profile of inflammatory factors in peripheral blood of control population and patients with fulminant myocarditis at admission. In the figure, control represents the control population, FM represents patients with fulminant myocarditis; True indicates that value is greater than threshold and difference is significant. False indicates that value is less than threshold and difference is not significant.
Figure 2 shows change profile of inflammatory factors in peripheral blood of patients with fulminant myocarditis before and after treatment. In the figure, FM-admission represents blood test results of patients with fulminant myocarditis when admitted without untreated. FM-discharge represents result of a blood sample test performed on patient with fulminant myocarditis after treatment and before discharge; True indicates that value is greater than threshold and difference is significant. False indicates that value is less than threshold and difference is not significant.
Figure 3 shows expression levels of specific inflammatory factors in patients with fulminant myocarditis. In the figure, FM-admission represents expression levels of inflammatory factors in blood samples of patients with fulminant myocarditis when admitted without treatment. FM-discharge represents expression level of inflammatory cytokines in blood sample of a patient with fulminant myocarditis after treatment and before discharge; control represents expression level of inflammatory factors in blood samples of control population; folds refers to a fold change value of expression level. Cytokines refer to inflammatory factors.

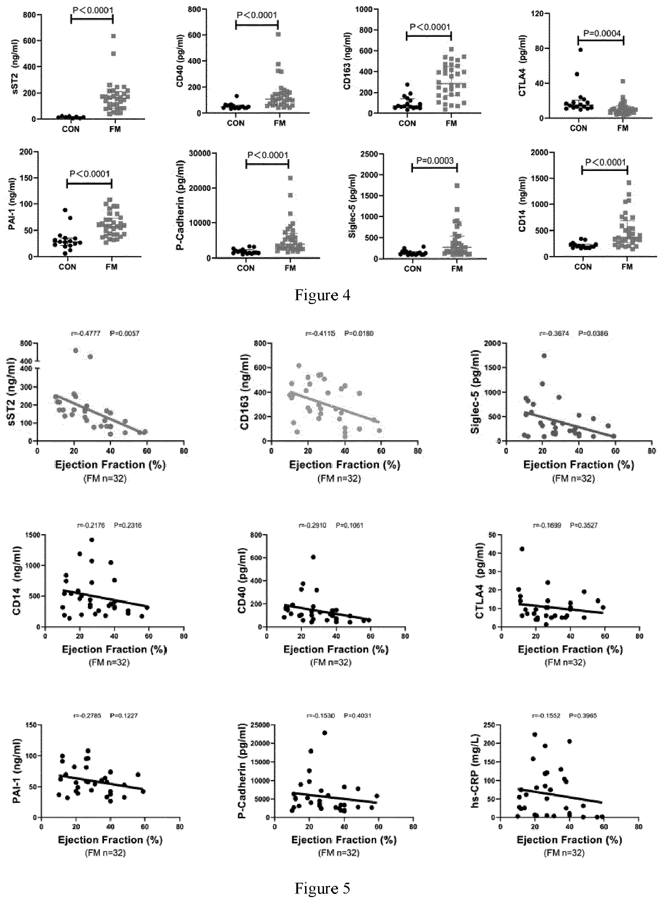

Figure 4 shows chart of content analysis on 8 inflammatory factors in blood samples of control population and patients with fulminant myocarditis, indicating that 8 inflammatory factors can be used in diagnosis and evaluation of fulminant myocarditis. In the figure, con represents expression level of inflammatory factors in blood samples of control population. FM represents expression level of inflammatory factors in blood samples from patients with fulminant myocarditis.
Figure 5 shows analysis of correlation between 9 inflammatory factors and cardiac ejection fraction, among which 3 inflammatory factors can be used to evaluate prognosis of fulminant myocarditis. In the figure, Ejection Fraction represents Ejection Fraction, and FM represents expression level of inflammatory factors in blood samples of patients with fulminant myocarditis; hs-CRP represents positive control.

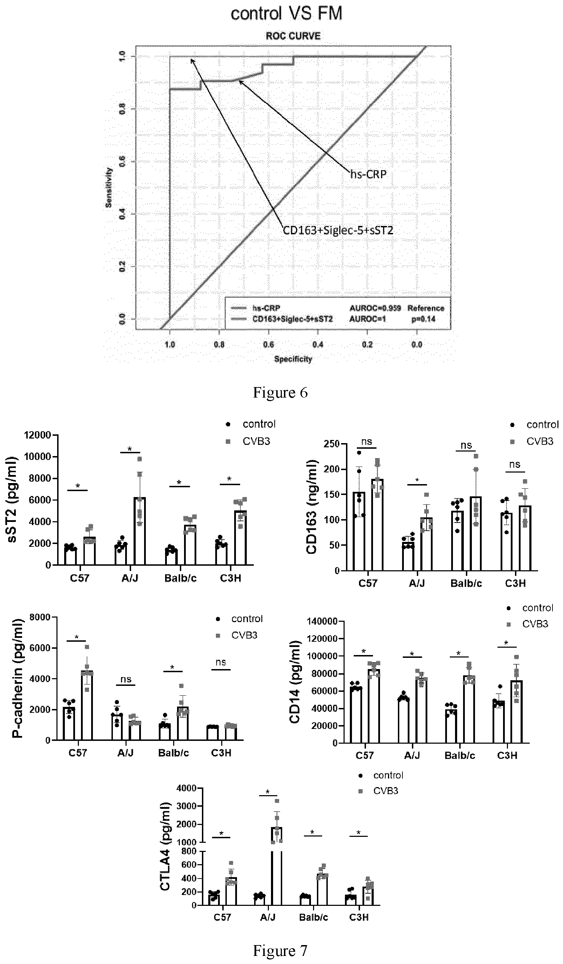

Figure 6 shows ROC curve of diagnostic efficacy of peripheral blood levels of specific inflammatory factors for fulminant myocarditis; in the figure, hs-CRP represents positive control.
Figure 7 shows bar chart of levels of inflammatory factors in four groups of mice in experiment example 4, indicating that levels of various inflammatory factors in peripheral blood of fulminant myocarditis A/J mice are increased. In the figure, C57 represents C57 mice, A/J represents A/J mice, Balb/c represents Balb/c mice, C3H represents C3H mice, Control represents blank control group, and CVB3 represents CVB3 myocarditis model group.

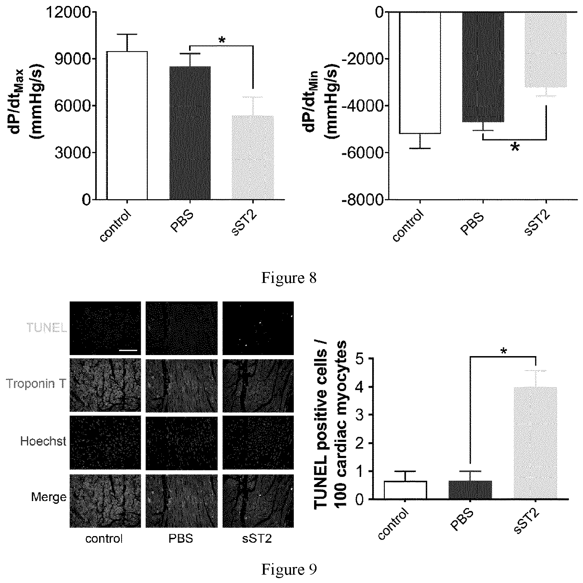

Figure 8 shows numerical bar graphs of maximum velocity of increase of left ventricular pressure (-dP/dtₘᵢₙ) and maximum velocity of decrease of left ventricular pressure(-dP/dtₘᵢₙ) of three groups of mice in experiment example 5, indicating that sST2 can induce cardiac dysfunction in A/J mice.
Figure 9 shows TUNEL staining of cardiomyocytes and bar chart of TUNEL positive cell rate per 100 cardiomyocytes (TUNEL positive rate) of 3 groups of mice in experiment example 5, indicating that sST2 can induce apoptosis of cardiomyocytes in A/J mice. In Figure 7 and Figure 8, control represents blank control group; PBS represents negative control group; sST2 represents experimental group.

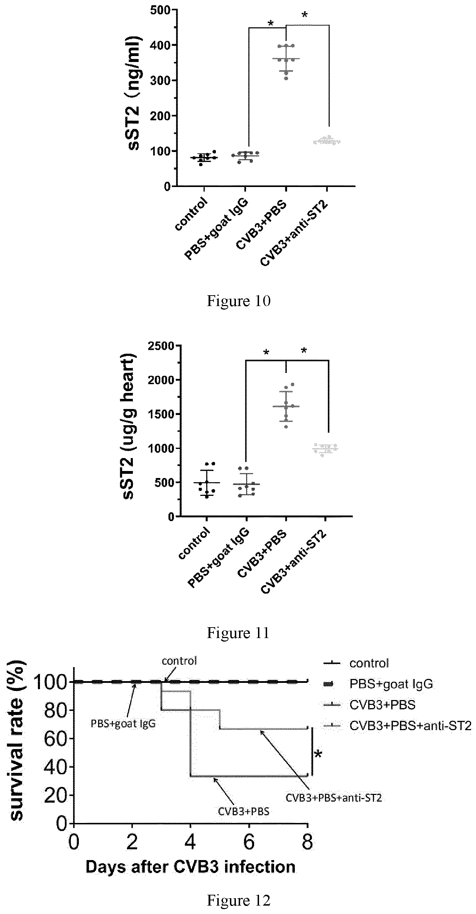

Figure 10 shows chart of analysis on content of sST2 in blood samples of mice in 4 groups of experimental example 6, indicating that sST2 neutralizing antibody can inhibit the increased sST2 in peripheral blood of mice with A/J fulminant myocarditis induced by CVB3.
Figure 11 shows chart of analysis on content of sST2 in myocardial tissue of 4 groups of mice in experiment example 6, indicating that sST2 neutralizing antibody can inhibit the increased sST2 in heart of mice with A/J fulminant myocarditis induced by CVB3.
Figure 12 shows chart of analysis on survival rate of 4 groups of mice in experiment example 6, indicating that sST2 neutralizing antibody can improve survival rate of A/J fulminant myocarditis mice induced by CVB3.

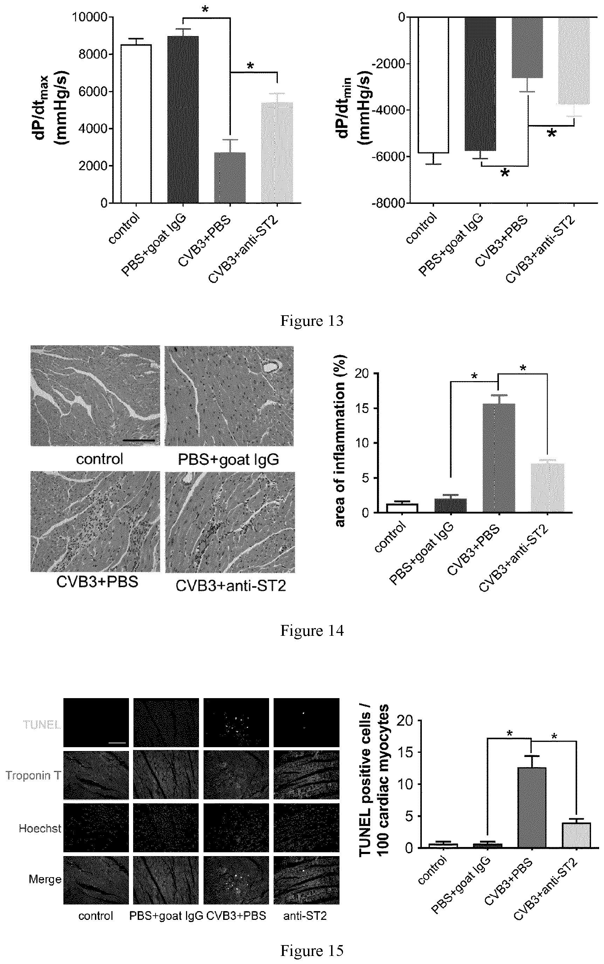

Figure 13 shows numerical bar graphs of maximum velocity of decrease of left ventricular pressure (-dP/dtₘᵢₙ) and maximum velocity of increase of left ventricular pressure(-dP/dtₘₐₓ) of 4 groups of mice in experiment example 6, indicating that sST2 neutralizing antibody can improve the damaged cardiac function of mice with A/J fulminant myocarditis induced by CVB3.
Figure 14 shows staining of myocardial tissue sections and histogram of proportion of inflammatory regions in myocardial tissue of 4 groups of mice in experiment example 6, indicating that sST2 neutralizing antibody can reduce inflammatory cell infiltration in heart of mice with A/J fulminant myocarditis induced by CVB3.
Figure 15 shows TUNEL staining of cardiomyocytes and bar chart of TUNEL positive cell rate per 100 cardiomyocytes (TUNEL positive rate) of 4 groups of mice in experiment example 6, indicating that sST2 neutralizing antibody can reduce cardiomyocyte apoptosis in heart of mice with A/J fulminant myocarditis induced by CVB3.
   In Figures 10-15, control represents blank control group; PBS+goat IgG represented negative control group. CVB3+PBS represented CVB3 fulminant myocarditis model group; CVB3+ anti-sST2 or anti-sST2 represented treatment group.

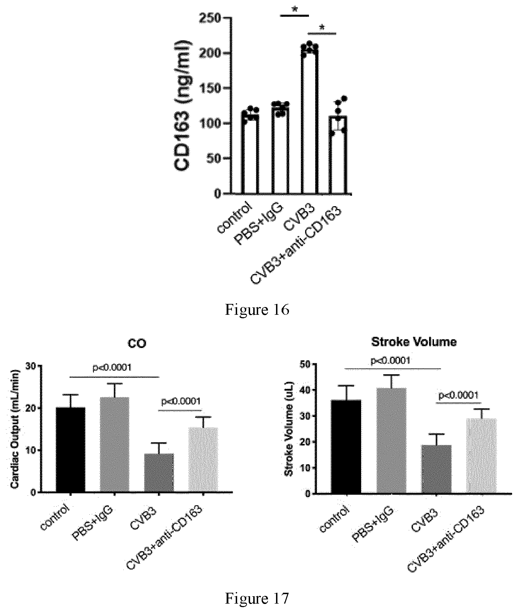

Figure 16 shows expression levels of inflammatory factors in peripheral blood of fulminant myocarditis mice in experimental example 7, suggesting that anti-CD163 neutralizing antibodies can inhibit increase of CD163. In the figure, ordinate is expression level of CD163 inflammatory factor, and on abscissa, control represents blank control group, PBS+IgG represents negative control group, CVB3 represents CVB3 fulminant myocarditis model group, and CVB3+ anti-CD163 represents treatment group.
Figure 17 shows cardiac function of mice in experimental example 7, indicating that anti-CD163 neutralizing antibody can improve cardiac dysfunction of A/J fulminant myocarditis mice induced by CVB3. In the figure, on the left ordinate, cardiac output represents the measured value of cardiac output, and on the right ordinate, stroke volume represents stroke output, control represented blank control group, PBS+IgG represented negative control group, CVB3 represented CVB3 fulminant myocarditis model group, and CVB3+ anti-CD163 represented treatment group.

### Embodiments

Technical solution of this present invention will be clearly and completely described in the light of the following specific embodiments. Clearly, the described embodiments are only a part of embodiments of this invention and not all of them. Based on embodiments of the present invention, all other embodiments obtained by a person skilled in the art without creative works will fall within the scope of protection of the present invention.

### Sources of biological materials

Mice used in the experimental examples of this invention can be purchased commercially.

### Examples Group 1, use of the biomarker combination of the invention

This group of examples provides use of biomarker or biomarkers combination in preparing diagnostic reagents for fulminant myocarditis, characterized in that, the biomarker comprises: Siglec-5.

The biomarkers combination is selected from the group consisting of sST2, PAI-1, Siglec-5, CD163, CD40, P-Cadherin, CD14, CTLA4.

Specifically, the biomarkers combination is selected from one or more of sST2, PAI-1, Siglec-5, CD163, CD40, P-Cadherin, CD14, CTLA4.

More specifically, the biomarkers combinations is selected from one, two, three, four, five, six, seven, or eight of sST2, PAI-1, Siglec-5, CD163, CD40, P-Cadherin, CD14, CTLA4.

The diagnostic reagents for fulminant myocarditis include the biomarkers combination and other diagnostically acceptable auxiliary reagents; the diagnostically acceptable auxiliary reagents include: ELISA test reagents, or flow detection reagents.

The diagnosis includes determining whether suffer from fulminant myocarditis, or assessing prognosis of fulminant myocarditis;
preferably, the diagnosis refers to whether suffer from fulminant myocarditis, or prognosis of fulminant myocarditis is assessed based on expression level of biomarker in the biomarkers combination in subject;
preferably, when Siglec-5 expression level ≥89.56 pg/mL,
and/or, sST2 expression level ≥21.39 ng/mL,
and/or, PAI-1expression level ≥41.29 ng/mL,
and/or, CD163 expression level ≥133.8 ng/mL,
and/or, CD40 expression level ≥67.43 pg/mL,
and/or, P-Cadherin expression level ≥2634 pg/mL,
and/or, CD14 expression level ≥262.4 ng/mL,
and/or, CTLA4 expression level ≥11.19 pg/mL, fulminant myocarditis is diagnosed;
preferably, when Siglec-5 expression level < 89.56 pg/mL,
and/or, sST2 expression level < 21.39 ng/mL,
and/or, CD163 expression level < 133.8 ng/mL, prognosis of fulminant myocarditis is good.

The fulminant myocarditis is selected from fulminant myocarditis caused or triggered by viruses, bacteria, allergies, drugs, upper respiratory tract infections, and/or, intestinal infection.

All above markers in this disclosure have conventional technical meanings commonly understood by a person skilled in the art, such as:
Siglec-5 can be Siglec-5 as described in Patent Application No. 201980062880.6;
sST2 can be the sST2 recorded in "Chinese Guidelines for Diagnosis and Treatment of Heart Failure";
PAI-1 can be the PAI-1 described in an article of "Research on Application of PAI-1 Gene Testing for Ischemic stroke Prevention";
CD 163 can be the CD 163 described in an article of "Research Progress of M2 macrophage marker CD163 and tumor";
CD40 can be the CD40 described in an article of "Research on correlation between serum soluble CD40 ligand levels and Disease Activity in Crohn's Disease";
P-Cadherin can be the P-Cadherin described in "Ultrasensitive Detection of Fn14, P-Cadherin and N-Cadherin Based on Single Molecule Array Technology";
CD14 can be the CD14 recorded in an article of "Research on correlation between CT signs of AIDS complicated with pulmonary tuberculosis and plasma soluble CD14";
CTLA4 can be the CTLA4 described in an article of "Correlation between serum GP73 and CTLA4 levels and degree of liver fibrosis in patients with chronic hepatitis B";
hs-CRP can be the hs-CRP described in an article of "Study on Relationship between serum PTX3, CAV-1, hs-CRP and condition and prognosis of patients with acute cerebral infarction".

Examples Group 2, pharmaceutical use of biomarkers combination of this invention.

Examples Group 2, pharmaceutical use of biomarkers combination of this invention

This group of examples provides use of biomarker or biomarkers combination in preparing drug to prevent and cure fulminant myocarditis, characterized in that, the biomarker comprises: Siglec-5.

The biomarkers combination is selected from the group consisting of sST2, PAI-1, Siglec-5, CD163, CD40, P-Cadherin, CD14, CTLA4.

Specifically, the biomarkers combination is selected from one or more of sST2, PAI-1, Siglec-5, CD163, CD40, P-Cadherin, CD14, CTLA4;

More specifically, the biomarkers combination is selected from one, two, three, four, five, six, seven, or eight of sST2, PAI-1, Siglec-5, CD163, CD40, P-Cadherin, CD14, CTLA4.

Drug target of the drug to prevent and cure fulminant myocarditis is biomarker in the biomarkers combination.

Preferably, the drug to prevent and cure fulminant myocarditis includes an active ingredient that reduces, downregulates, or inhibits the biomarker.

Preferably, the active ingredient that reduces, downregulates or inhibits the biomarker is selected from inhibitor, neutralizing antibody or polypeptide of the biomarker.

The drug to prevent and cure fulminant myocarditis takes the biomarker in the biomarkers combination as the drug target, and achieves the effect of preventing and curing fulminant myocarditis by inhibiting expression level of the biomarker.

Preferably, the drug to prevent and cure fulminant myocarditis achieves effect of preventing and curing fulminant myocarditis by reducing expression level of biomarker in plasma of patients with fulminant myocarditis by inhibitor, neutralizing antibody or peptide against the biomarker. Preferably, the fulminant myocarditis is defined as fulminant myocarditis caused or triggered by viruses, bacteria, allergies, drugs, upper respiratory tract infections, and/or intestinal infections. The drug to prevent and cure fulminant myocarditis also includes pharmaceutically acceptable excipients.

Preferably, the pharmaceutically acceptable excipients include: solvent, propellant, solubilizer, cosolvent, emulsifying agent, colorant, adhesive, disintegrant, filling agent, lubricant, wetting agent, osmotic pressure regulator, stabilizer, flow aid, flavoring agent, preservative, suspending agent, coating material, aromatic, anti-adhesive, integrator, osmosis enhancer, pH regulator, buffering agent, plasticizer, surface active agent, foaming agent, elimination foaming agent, thickening agent, inclusion agent, humectant, absorbent, diluent, flocculant, deflocculant, filter aid, release blocker.

### The experimental example 1

### Expression levels of multiple inflammatory factors in peripheral blood of patients with fulminant myocarditis are changed

Study population and study design: From April 2017 to March 2019, consecutive inpatients with fulminant myocarditis were enrolled in Tongji Hospital, Tongji Medical College, Huazhong University of Science and Technology, Wuhan, Hubei Province. The inclusion criteria included: CD older than 18 years; ②Diagnosis of fulminant myocarditis: myocardial biopsy suggests myocarditis or clinical diagnosis (rapid onset of severe hemodynamic disturbance symptoms, increased cTnI, cardiac ultrasound suggested rapid reduction of left ventricular EF value or less than 40%). Exclusion criteria included: CD acute myocardial infarction; ② common acute myocarditis; ③ severe neurological diseases (Alzheimer's disease, progressive stage of Parkinson's syndrome), lower limb disability or deaf-mute patients.

Detection of inflammatory factors expression profile: fasting peripheral blood was collected into EDTA anticoagulant tube, and serum was isolated immediately by centrifugation at 3000 g for 8 minutes and then frozen at -80°C until analysis. Plasma inflammatory factors profile was detected by Human Cytokine Arrays (RayBiotech Company).

Experimental results: 4 patients with fulminant myocarditis were enrolled in this study, and 4 people with matched baseline data including gender, age and etc., were enrolled in control group. The results showed that compared with the control group, levels of 58 inflammatory factors in peripheral blood of patients with fulminant myocarditis were significantly changed (figure 1). These results suggested that changed levels of numerous inflammatory factors in plasma were associated with fulminant myocarditis. In the figure, control represents the control group population, FM represents patients with fulminant myocarditis, and red dots represent inflammatory factors with significant differences.

### The experimental example 2

### Expression level of specific inflammatory factors in peripheral blood of patients with fulminant myocarditis were back to normal level after treatment and improved

Study population and study design: From April 2017 to March 2019, consecutive inpatients with fulminant myocarditis were enrolled in Tongji Hospital, Tongji Medical College, Huazhong University of Science and Technology, Wuhan, Hubei Province. The inclusion criteria included: CD older than 18 years; ②Diagnosis of fulminant myocarditis: myocardial biopsy suggested myocarditis or clinical diagnosis (rapid onset of severe hemodynamic disturbance symptoms, increased cTnI, cardiac ultrasound suggested rapid reduction of left ventricular EF value or less than 40%). Exclusion criteria included: ① acute myocardial infarction; ② common acute myocarditis; ③ severe neurological diseases (Alzheimer's disease, progressive stage of Parkinson's syndrome), lower limb disability or deaf-mute patients.

Detection of inflammatory factor expression profile: fasting peripheral blood was collected into EDTA anticoagulant tube, and serum was isolated immediately by centrifugation at 3000 g for 8 minutes and then frozen at -80°C until analysis. Plasma inflammatory factor expression profile was detected by Human Cytokine Arrays (RayBiotech Company).

Experimental results: 4 patients with fulminant myocarditis were enrolled in this study, blood samples were collected at admission without treatment and after treatment as well as improved before discharge. The results showed that after effective treatment, content of some changed inflammatory factors significantly returned (figure 2). These results suggested that the changed levels of specific inflammatory factors in peripheral blood were associated with fulminant myocarditis, namely sST2, PAI-1, Siglec-5, CD163, IL-17B, IL-4, VEGF-C, CD40, P-Cadherin, CD14, Angiostatin and CTLA4 (figure 3). ^{∗} means p<0.05.

### The experimental example 3

### Levels of specific inflammatory factors in peripheral blood for diagnosis of fulminant myocarditis and prognostic evaluation of fulminant myocarditis

Study population and study design: From April 2017 to March 2019, consecutive inpatients with fulminant myocarditis were enrolled in Tongji Hospital, Tongji Medical College, Huazhong University of Science and Technology, Wuhan, Hubei Province. The inclusion criteria included: ① older than 18 years; ②Diagnosis of fulminant myocarditis: myocardial biopsy suggested myocarditis or clinical diagnosis (rapid onset of severe hemodynamic disturbance symptoms, increased cTnI, cardiac ultrasound suggested rapid reduction of left ventricular EF value or less than 40%). Exclusion criteria included: ① acute myocardial infarction; ② common acute myocarditis; ③ severe neurological diseases (Alzheimer's disease, progressive stage of Parkinson's syndrome), lower limb disability or deaf-mute patients.

Detection of inflammatory factor levels: fasting peripheral blood was collected into EDTA anticoagulant tube, and serum was isolated immediately by centrifugation at 3000 g for 8 minutes and then frozen at -80°C until analysis. Plasma inflammatory factor level was detected by ELISA kit (R&D Company).

Experimental results: 32 patients with fulminant myocarditis were enrolled in this study, and 16 patients whose matching baseline data like gender, age and etc., has no difference with 4 patients were enrolled in control group. The results showed that levels of 8 inflammatory factors (sST2, PAI-1, Siglec-5, CD163, CD40, P-Cadherin, CD14 and CTLA4) in peripheral blood of patients with fulminant myocarditis were significantly changed, which had diagnostic value (figure 4). Among them, levels of 3 inflammatory factors (sST2, Siglec-5 and CD163) were correlated with EF values of cardiac function, which had prognostic value (figure 5). Moreover, levels of specific inflammatory factors (sST2, Siglec-5 and CD163) in peripheral blood of patients had better diagnostic efficacy than CRP (Figure 6). ^{∗} represents p<0.05.

### The experimental example 4

### Levels of inflammatory factors in peripheral blood of fulminant myocarditis mice increased

Mouse model preparation: 8-week-old male C57, A/J, Balb/c and C3H mice were purchased from Model Animal Center of Nanjing University, and were raised in SPF animal room of Tongji Medical College, Huazhong University of Science and Technology. Mice with different backgrounds were intraperitoneally injected with CVB3 virus (10⁴ dissolved in 0.1ml PBS), respectively, after one week of adaptation in the animal house, and used as model group of CVB3 myocarditis.

Level of inflammatory factors was detected by ELISA Kit of R&D company.

Experimental results: cardiac catheter detection was performed 8 days after intraperitoneal injection of CVB3 virus, and then animals were sacrificed to collect tissue samples for above detection. The results showed that sST2, C163, P-Cadherin, CD14 and CTLA4 were most significantly increased in peripheral blood of mice with A/J myocarditis after CVB3 infection compared with control group (FIG. 7). ^{∗} represents p<0.05.

### The experimental example 5

### Taking sST2 as an example, effects of certain inflammatory factors on cardiac function of A/J mice were observed

Mouse model preparation: 8-week-old male A/J mice were purchased from the Model Animal Center of Nanjing University, and were raised in SPF animal room of Tongji Medical College, Huazhong University of Science and Technology. Mice were intraperitoneally injected with recombinant sST2 protein (5ug dissolved in 0.1ml PBS) one week after adaptation to the animal room, as intervention group (experimental group). Another group of mice were treated with PBS as negative control group, and the third group was blank control group without any treatment (control).

Cardiac hemodynamic assays in mice were performed by using the Millar Pressure-volume system of Millar Instrument PowerLab Company. After animals were anesthetized to a proper depth, a median incision was made in their neck, the right common carotid artery was separated, its distal end was ligated, and the proximal end was clipped. A V-shaped incision was made in artery with microscissors, and a microcatheter was inserted until the left ventricle, and signals were recorded through conduction system. Hemodynamic data such as heart rate (HR), left ventricular end-diastolic pressure (PED), left ventricular end-systolic pressure (PES), maximum rate of decline of left ventricular pressure (-dP/dtₘᵢₙ) and maximum rate of rise of left ventricular pressure (+dP/dtₘₐₓ) were obtained.

Histological detection: myocardial tissue was immersed and fixed in neutral formaldehyde solution in the embedding frame, dehydrated, embedded in paraffin to obtain paraffin block. The paraffin block was cut into sections with a thickness of 4µm on microtome. HE staining was used to observe gross appearance of myocardium, and TUNEL staining was detected by test kit of Roche Company.

Experimental results: cardiac catheter detection was performed 10 days after intraperitoneal injection of recombinant sST2 protein, and then the animals were sacrificed and tissue samples were collected for above detection. The results showed that intraperitoneal injection of recombinant sST2 protein significantly decreased cardiac function of mice compared with control group (figure 8). Moreover, intraperitoneal injection of recombinant sST2 protein increased TUNEL positive rate in cardiomyocytes, suggesting increased apoptosis of cardiomyocytes (figure 9). ^{∗} represents p<0.05.

### The experimental example 6

### Anti-sST2 neutralizing antibody can inhibit myocardial injury induced by CVB3 in A/J fulminant myocarditis mice

Mouse model preparation: 8-week-old male A/J mice were purchased from the Model Animal Center of Nanjing University, and were raised in SPF animal room of Tongji Medical College, Huazhong University of Science and Technology. Mice were given intraperitoneal injection of CVB3 virus (10⁴ dissolved in 0.1ml PBS) one week after acclimation to the animal room as model group of fulminant myocarditis of CVB3. Another group of fulminant myocarditis model mice were intraperitoneally injected with anti-sST2 neutralizing antibody (10µg dissolved in 0.1ml PBS) as treatment group. The third group was blank control group without any treatment. The fourth group was negative control group which received intraperitoneal injection of goat IgG antibody (10µg dissolved in 0.1ml PBS).

sST2 levels were detected by Mouse ST2/IL-33R Quantikine ELISA Kit of R&D company.

Cardiac hemodynamic assays in mice were performed using the Millar Pressure-volume system from Millar Instrument PowerLab Company. After animals were anesthetized to a proper depth, a median incision was made in their neck, the right common carotid artery was separated, its distal end was ligated, and the proximal end was clipped. A V-shaped incision was made in artery with microscissors, and a microcatheter was inserted until the left ventricle, and signals were recorded through conduction system. Hemodynamic data such as heart rate (HR), left ventricular end-diastolic pressure (PED), left ventricular end-systolic pressure (PES), maximum rate of decline of left ventricular pressure (-dP/dtₘᵢₙ) and maximum rate of rise of left ventricular pressure (+dP/dtₘₐₓ) were obtained.

Histological detection: myocardial tissue was immersed and fixed in neutral formaldehyde solution in the embedding frame, dehydrated, embedded in paraffin to obtain paraffin block. The paraffin block was cut into sections with a thickness of 4µm on microtome. HE staining was used to observe gross appearance of myocardium, and TUNEL staining was detected by test kit of Roche Company.

Experimental results: cardiac catheter detection was performed 8 days after intraperitoneal injection of anti-sST2 neutralizing antibody, and then the animals were sacrificed and tissue samples were collected for above detection. The results showed that sST2 in peripheral blood of mice with fulminant myocarditis after CVB3 infection increased compared with control group, while anti-sST2 neutralizing antibody could reduce it (figure 10). sST2 was increased in heart of mice with fulminant myocarditis after CVB3 infection, which was decreased by anti-sST2 neutralizing antibodies (figure 11). Survival rate of fulminant myocarditis mice after CVB3 infection was significantly decreased, which was increased by anti-sST2 neutralizing antibody (figure 12). Cardiac function of fulminant myocarditis mice after CVB3 infection was significantly decreased, which was enhanced by anti-sST2 neutralizing antibodies (figure 13). Inflammatory cell infiltration was significantly increased in heart of fulminant myocarditis mice after CVB3 infection, which was reduced by anti-sST2 neutralizing antibody (figure 14). TUNEL positive rate in cardiomyocytes of mice with fulminant myocarditis after CVB3 infection was significantly increased, which was reduced by anti-sST2 neutralizing antibody (figure 15). ^{∗} represents p<0.05.

### The experimental example 7

### Anti-CD163 neutralizing antibody can improve cardiac dysfunction in A/J fulminant myocarditis mice induced by CVB3

Mouse model preparation: 8-week-old male A/J mice were purchased from the Model Animal Center of Nanjing University, and were raised in SPF animal room of Tongji Medical College, Huazhong University of Science and Technology. Mice were given intraperitoneal injection of CVB3 virus (10⁴ dissolved in 0.1ml PBS) one week after acclimation to the animal room as model group of fulminant myocarditis of CVB3. Mice without any treatment were used as blank control group, and mice with goat IgG antibody (10µg dissolved in 0.1ml PBS) injected intraperitoneally were used as negative control group. In addition, mice in fulminant myocarditis model group were intraperitoneally injected with anti-CD163 neutralizing antibody (10µg dissolved in 0.1 ml PBS) as treatment group.

Level of inflammatory factors was detected by ELISA Kit of R&D company.

Mouse cardiac ultrasound testing: cardiac ultrasound was performed by using Vevo 2100 Small animal ultrasound of Visual Sonics Inc. Main measuring indicators included heart rate (HR), left ventricular ejection fraction (LVEF), left ventricular short-axis shortening rate (LVFS), left ventricular septal thickness during diastole or systole (LVIDd and LVIDs), and left ventricular posterior wall thickness (LVPWd and LVPWs).

Experimental results: cardiac catheter detection was performed 8 days after intraperitoneal injection of anti-CD163 neutralizing antibody. The animals were sacrificed and tissue samples were collected for above detection. The results showed that CD163 level in peripheral blood of fulminant myocarditis mice after CVB3 infection was increased compared with control group. However, treatment with anti-CD163 neutralizing antibodies decreased CD163 levels (Figure 16). Cardiac function was significantly decreased in fulminant myocarditis mice after CVB3 infection, which was enhanced by anti-CD163 neutralizing antibody treatment (figure 17). ^{∗} represents p<0.05.

The invention further relates to the following items:
1. Use of biomarker or biomarkers combination in preparing diagnostic reagents for fulminant myocarditis, characterized in that, the biomarker comprises: Siglec-5.
2. The use of biomarker or biomarkers combination in preparing diagnostic reagents for fulminant myocarditis of item 1, characterized in that, the biomarkers combination is selected from the group consisting of sST2, PAI-1, Siglec-5, CD163, CD40, P-Cadherin, CD14, CTLA4.
3. The use of biomarker or biomarkers combination in preparing diagnostic reagents for fulminant myocarditis of item 1 or 2, characterized in that, the diagnostic reagents for fulminant myocarditis includes the biomarkers combination and other diagnostically acceptable auxiliary reagents; the diagnostically acceptable auxiliary reagents include: ELISA test reagents, or flow detection reagents.
4. The use of biomarker or biomarkers combination in preparing diagnostic reagents for fulminant myocarditis of any of items 1-3, characterized in that, the diagnosis includes determining whether suffer from fulminant myocarditis, or assessing prognosis of fulminant myocarditis;
   and/or, the diagnosis refers to whether suffer from fulminant myocarditis, or prognosis of fulminant myocarditis is assessed based on expression level of biomarker in the biomarkers combination in subject;
   and/or, when Siglec-5 expression level ≥89.56 pg/mL,
   and/or, sST2 expression level ≥21.39 ng/mL,
   and/or, PAI-1expression level ≥41.29 ng/mL,
   and/or, CD163 expression level ≥133.8 ng/mL,
   and/or, CD40 expression level ≥67.43 pg/mL,
   and/or, P-Cadherin expression level ≥2634 pg/mL,
   and/or, CD14 expression level ≥262.4 ng/mL,
   and/or, CTLA4 expression level ≥11.19 pg/mL, fulminant myocarditis is diagnosed;
   and/or, when Siglec-5 expression level < 89.56 pg/mL,
   and/or, sST2 expression level < 21.39 ng/mL,
   and/or, CD163 expression level < 133.8 ng/mL, the prognosis of fulminant myocarditis is good.
5. The use of biomarker or biomarkers combination in preparing diagnostic reagents for fulminant myocarditis in any of items 1-4, characterized in that, the fulminant myocarditis is selected from fulminant myocarditis caused or triggered by viruses, bacteria, allergies, drugs, upper respiratory tract infections, and/or, intestinal infection.
6. Use of biomarker or biomarkers combination in preparing drug to prevent and cure fulminant myocarditis, characterized in that, the biomarker comprises: Siglec-5.
7. The use of biomarker or biomarkers combination in preparing drug to prevent and cure fulminant myocarditis of item 6, characterized in that, the biomarkers combination is selected from the group consisting of sST2, PAI-1, Siglec-5, CD163, CD40, P-Cadherin, CD14, CTLA4.
8. The use of biomarker or biomarkers combination in preparing drug to prevent and cure fulminant myocarditis of item 6 or 7, characterized in that, drug target of the drug to prevent and cure fulminant myocarditis is biomarker in the biomarkers combination.
   and/or, the drug to prevent and cure fulminant myocarditis includes an active ingredient that reduces, downregulates, or inhibits the biomarker;
   and/or, the active ingredient that reduces, downregulates or inhibits the biomarker is selected from inhibitor, neutralizing antibody or polypeptide of the biomarker.
9. The use of biomarker or biomarkers combination in preparing drug to prevent and cure fulminant myocarditis of any of items 6-8, characterized in that, the drug to prevent and cure fulminant myocarditis takes the biomarker in the biomarkers combination as the drug target, and achieves the effect of preventing and curing fulminant myocarditis by inhibiting expression level of the biomarker;
   and/or, the drug to prevent and cure fulminant myocarditis achieves effect of preventing and curing fulminant myocarditis by reducing expression level of biomarker in plasma of patients with fulminant myocarditis by inhibitor, neutralizing antibody or peptide against the biomarker;
   and/or, the fulminant myocarditis is defined as fulminant myocarditis caused or triggered by viruses, bacteria, allergies, drugs, upper respiratory tract infections, and/or intestinal infections.
10. The use of biomarker or biomarkers combination in preparing drug to prevent and cure fulminant myocarditis of any of items 6-9, characterized in that, the drug to prevent and cure fulminant myocarditis also includes pharmaceutically acceptable excipients.

## Claims

1. Use of biomarker or biomarkers combination in preparing diagnostic reagents for fulminant myocarditis, **characterized in that**, the biomarker comprises: Siglec-5.

2. The use of biomarker or biomarkers combination in preparing diagnostic reagents for fulminant myocarditis of claim 1, **characterized in that**, the biomarkers combination is selected from the group consisting of sST2, PAI-1, Siglec-5, CD163, CD40, P-Cadherin, CD14, CTLA4.

3. The use of biomarker or biomarkers combination in preparing diagnostic reagents for fulminant myocarditis of claim 1 or 2, **characterized in that**, the diagnostic reagents for fulminant myocarditis includes the biomarkers combination and other diagnostically acceptable auxiliary reagents; the diagnostically acceptable auxiliary reagents include: ELISA test reagents, or flow detection reagents.

4. The use of biomarker or biomarkers combination in preparing diagnostic reagents for fulminant myocarditis of any of claims 1-3, **characterized in that**, the diagnosis includes determining whether suffer from fulminant myocarditis, or assessing prognosis of fulminant myocarditis;
and/or, the diagnosis refers to whether suffer from fulminant myocarditis, or prognosis of fulminant myocarditis is assessed based on expression level of biomarker in the biomarkers combination in subject;
and/or, when Siglec-5 expression level ≥89.56 pg/mL,
and/or, sST2 expression level ≥21.39 ng/mL,
and/or, PAI-1 expression level ≥41.29 ng/mL,
and/or, CD163 expression level ≥133.8 ng/mL,
and/or, CD40 expression level ≥67.43 pg/mL,
and/or, P-Cadherin expression level ≥2634 pg/mL,
and/or, CD14 expression level ≥262.4 ng/mL,
and/or, CTLA4 expression level ≥11.19 pg/mL, fulminant myocarditis is diagnosed;
and/or, when Siglec-5 expression level < 89.56 pg/mL,
and/or, sST2 expression level < 21.39 ng/mL,
and/or, CD163 expression level < 133.8 ng/mL, the prognosis of fulminant myocarditis is good.

5. The use of biomarker or biomarkers combination in preparing diagnostic reagents for fulminant myocarditis in any of claims 1-4, **characterized in that**, the fulminant myocarditis is selected from fulminant myocarditis caused or triggered by viruses, bacteria, allergies, drugs, upper respiratory tract infections, and/or, intestinal infection.

6. A biomarker or a combination of biomarkers for use in a medical method for preventing and/or curing fulminant myocarditis, **characterized in that**, the biomarker comprises: Siglec-5.

7. The biomarker or the combination of biomarkers for use according to claim 6, **characterized in that** the combination of biomarkers is selected from the group consisting of sST2, PAI-1, Siglec-5, CD163, CD40, P-Cadherin, CD14, CTLA4.

8. The biomarker or the combination of biomarkers for use according to claim 6 or 7, **characterized in that** the drug target of the drug to prevent and/or cure fulminant myocarditis is a biomarker in the combination of biomarkers;
and/or, the drug to prevent and/or cure fulminant myocarditis includes an active ingredient that reduces, downregulates, or inhibits the biomarker;
and/or, the active ingredient that reduces, downregulates or inhibits the biomarker is selected from an inhibitor, a neutralizing antibody or a polypeptide of the biomarker.

9. The biomarker or the combination of biomarkers for use according to any of claims 6-8, **characterized in that** the drug to prevent and/or cure fulminant myocarditis takes the biomarker in the biomarkers combination as the drug target, and achieves the effect of preventing and/or curing fulminant myocarditis by inhibiting the expression level of the biomarker;
and/or, the drug to prevent and/or cure fulminant myocarditis achieves the effect of preventing and/or curing fulminant myocarditis by reducing the expression level of the biomarker in the plasma of patients with fulminant myocarditis by being an inhibitor, a neutralizing antibody or a peptide against the biomarker;
and/or, the fulminant myocarditis is defined as fulminant myocarditis caused or triggered by viruses, bacteria, allergies, drugs, upper respiratory tract infections, and/or intestinal infections.

10. The biomarker or the combination of biomarkers for use according to any of claims 6-9, **characterized in that** the drug to prevent and/or cure fulminant myocarditis also includes pharmaceutically acceptable excipients.
